(19) Europäisches Patentamt

European Patent Office

Office européen des brevets

(11) EP 0 715 848 A2

(12) EUROPÄISCHE PATENTANMELDUNG

(43) Veröffentlichungstag:
12.06.1996 Patentblatt 1996/24

(51) Int. Cl.$^6$: **A61K 9/20**

(21) Anmeldenummer: 95118420.9

(22) Anmeldetag: 23.11.1995

(84) Benannte Vertragsstaaten:
CH DE FR GB IT LI

(30) Priorität: 06.12.1994 DE 4443358

(71) Anmelder: BASF Aktiengesellschaft
D-67063 Ludwigshafen (DE)

(72) Erfinder:
• Einig, Heinz, Dr.
D-67433 Neustadt (DE)
• Lang, Siegfried, Dr.
D-67071 Ludwigshafen (DE)

(54) **Depot-Arzneimittel enthaltend Alginat und polymeres Bindemittel**

(57) Depot-Arzneimittel, enthaltend einen Wirkstoff, Alginat und als Hilfsstoff mindestens ein wasserlösliches, polymeres Bindemittel aus der Gruppe Polyvinylpyrrolidon und Vinylacetat-Vinylpyrrolidon-Copolymere.

**Beschreibung**

Die vorliegende Erfindung betrifft ein Depot-Arzneimittel enthaltend einen Pharmawirkstoff, Alginat und ein wasserlösliches polymeres Bindemittel.

Depot-Arzneimittel mit einer bestimmten Freisetzungsrate des Wirkstoffs sind bekannt (WO 90/00391). Die Freisetzungsrate wird dabei vor allem durch das eingesetzte Alginat bestimmt. Aus der US 4 792 452 sind Depot-Arzneimittel bekannt, die einen Wirkstoff, ein Alignat, ein Quellungsmittel (u.a. Celluloseether) und ein Bindemittel (u. a. Lactose, auch Polyvinylpyrrolidon ist genannt) enthalten. Die Freisetzungsrate dieser Arzneimittel ist unabhängig vom pH-Wert der Umgebung.

Die bekannten Wirkstoff-enthaltenden Depot-Arzneimittel werden üblicherweise in Tablettenform hergestellt. Dabei tritt das Problem auf, daß die Tabletten bei der Herstellung und der Verpackung einer Abriebbeanspruchung ausgesetzt sind. Außerdem zeigt sich, daß die Oberfläche der Tabletten oft nicht ausreichend glatt ist, was zum einen einen nachteiligen optischen Eindruck ("Apfelsinenhaut") vermittelt, was aber zum anderen auch dazu führt, daß Coatingschichten schlechter aufgetragen werden können.

Der vorliegenden Erfindung lag daher die Aufgabe zugrunde, ein Depot-Arzneimittel in Tablettenform zu entwickeln, das einen möglichst geringen Abrieb, d.h. eine möglichst große Härte und Bruchfestigkeit aufweist und das sich außerdem gut beschichten läßt.

Diese Aufgabe wird gelöst durch ein Depot-Arzneimittel in Tablettenform, enthaltend einen Wirkstoff, Alginat und Hilfsstoffe, die dadurch gekennzeichnet sind, daß es als einen Hilfsstoff mindestens ein wasserlösliches, polymeres Bindemittel aus der Gruppe Polyvinyl-pyrrolidon und Vinylacetat-Vinylpyrrolidon-Copolymeres enthält, wobei das Bindemittel folgende Eigenschaften aufweist:

a) einen $M_w/M_n$-Wert von 1,5 - 5,0
b) Korngrößenverteilung des Bindemittelpulvers vor der Tablettierung:
Anteil < 50 µm : max. 18 %
Anteil > 250 µm : max. 20 %.

Es hat sich herausgestellt, daß Bindemittel, die diese Eigenschaften aufweisen, besonders dazu geeignet sind, die oben genannte Aufgabe zu lösen. Es ist anzunehmen, daß sich der $M_w/M_n$-Wert (Molmassenverteilung, Uneinheitlichkeitsfaktor) in dem genannten Bereich günstig auf die Härte bzw. Bruchfestigkeit und damit auf den Abrieb (Friabilität) auswirkt, während die spezielle Korngrößenverteilung möglicherweise die Oberflächeneigenschaften vorteilhaft beeinflußt.

Anzumerken ist, daß die Freisetzungscharakteristik mit dem neuen Depot-Arnzeimittel ebenso wie bei den in der WO 90/00391 beschriebenen Arzneimitteln sehr gut reproduzierbar eingestellt werden kann, obwohl die erwähnten vorteilhaften Effekte bezüglich des Abriebs und der Oberflächenbeschaffenheit erzielt werden.

Vorteilhafterweise enthalten die erfindungsgemäßen Depot-Arzneimittel als Bindemittel Polyvinylpyrrolidon und/oder VinylacetatVinylpyrrolidon-Copolymere mit einem $M_w/M_n$-Wert von 2,0 bis 4,5, ganz bevorzugt 2,5 bis 4,0. Insbesondere wird ein Vinylacetat-Vinylpyrrolidon-Copolymer mit 50 bis 70 Gew.-% Vinylpyrrolidon und 30 bis 50 Gew.-% Vinylacetat eingesetzt. Die Molekulargewichte der wasserlöslichen polymeren Bindemittel betragen bevorzugt 5.000 bis 1.500.000, insbesondere 10.000 bis 80.000, wobei die $M_w$-Werte für die Homopolymeren im allgemeinen bei 20.000 bis 1.500.000 und für die Copolymeren bei 5.000 bis 80.000 liegen.

Die Korngrößenverteilung (bestimmt im Luftstrahlsieb, 5 min, 20 mbar) beträgt bevorzugt max. 17 % <50 µm und max. 10 % >250 µm.

Die Bindemittelmenge in der Tablette kann 2 bis 50 Gew.-%, bevorzugt 4 bis 40 Gew.-% bezogen auf das Gesamtgewicht der Tablette, betragen.

Das Gewichtsverhältnis von Wirkstoff, Alginat und Bindemittel in dem Depot-Arzneimittel beträgt vorzugsweise 1 : 0,7-3 : 0,05-2,0.

Nach einer bevorzugten Ausführungsform hat das Depot-Arzneimittel die Form einer Filmtablette.

Als Alginate kommen beispielsweise Ammoniumalginat, Kaliumalginat, Natriumalginat oder Mischungen aus diesen Alginaten in Betracht. Bevorzugt ist Natriumalginat.

Das Alginat weist eine Viskosität in einer 1 %igen wäßrigen Lösung innerhalb eines Bereiches von x ± 10 mPa · s bei 20°C auf, worin x ein Wert von 20 bis 500 ist.

Der beanspruchte Viskositätsbereich bezieht sich auf die Messung der Viskosität mit einem Hake-Viskosimeter (Modell Rheomat® 30) bzw. mit einem Brookfield-Viskosimeter (Modell DV2). Mißt man die Viskosität mit anderen Geräten, so kann sich der Viskositätsbereich verschieben. Weiter ist zu beachten, daß bei der Herstellung der 1 %igen wäßrigen Alginatlösung für die Bestimmung der Viskosität der 1 %-ige Alginatanteil auf getrocknetes Alginat berechnet wird.

Die Herstellung der Alginate ist bekannt (vgl. Ullmanns Encyklopädie der technischen Chemie, 4. Auflage, Band 19, Seiten 245 bis 246 und darin zitierte Literatur; W.H. McNeely, D.J. Pettitt in Whistler: Industrial Gums, 2. Auflage, Academic Press, New York, Seite 49 ff).

Bevorzugt verwendet man solche Alginate, in denen das Verhältnis von Manuron- zu Guluronsäure zwischen 7:3 und 3:7, vorzugsweise zwischen 6:4 und 4:6, liegt. Diese lassen sich z.B. aus der Alge Laminaria hyperborea gewinnen.

Die erfindungsgemäßen Depot-Arzneimittel enthalten als Wirkstoff bevorzugt Verapamil, insbesondere als Säureadditionssalz, z.B. als Hydrochlorid.

Als Pharmawirkstoff sind jedoch auch andere oral verabreichbare Arzneistoffe einsetzbar. Diese umfassen z.B. Adrenergika, wie Salze von Ephedrin, Desoxyephedrin, Phenylephrin, Epinephrin, Salbutamol und Terbutalin; Cholinergika, wie Salze von Physostigmin und Neostigmin; Spasmolytika, wie Salze von Atropin, Methanthelin und Papaverin; Curarimimetika, wie Salze von Chlorisondamin, Sedativa und Muskelrelaxantien, wie Salze von Fluphenazin, Thioridazin, Trifluorperazin, Chlorpromazin und Triflupromazin; Antidepressivia, wie Salze von Amitriptylin und Nortriptylin; Antihistaminika, wie Salze von Diphenhydramin, Chlorpheniramin, Dimenhydrinat, Tripelenamin, Perphenazin, Chlorprophenazin und Chlorprophenpyridamin; Herzmittel, wie Salze von Verapamil, Diltiazem, Gallapomil, Cinnarizin, Propanolol, Metroprolol und Nadolol und Salze eines der nachstehenden Arzneistoffe: Antimalariamittel, wie Chlorochinin, Analgetika, wie Propoxyphen und Meperidin. Andere Arzneistoffe mit gleichen oder unterschiedlichen pharmakologischen Wirkungen können auch für das erfindungsgemäße Arzneimittel verwendet werden.

Die als Bindemittel in Betracht kommenden Polyvinylpyrrolidone werden durch radikalische Polymerisation von N-Vinylpyrrolidon hergestellt. Sie sind in Wasser, aber auch in vielen organischen Lösungsmitteln, wie z.B. in verschiedenen Alkoholen löslich und weisen Schüttdichten (DIN 53 468) von 300 bis 650 g/l auf.

Die als Bindemittel ebenfalls einsetzbaren Copolymeren aus N-Vinylpyrrolidon und Vinylacetat sind ebenfalls wasserlöslich (Löslichkeit von mehr als 10 %) und werden auf an sich bekannte Weise hergestellt.

Sichergestellt sein muß die genannte Molmassenverteilung, wobei die der Angabe zugrunde liegenden $M_w$- und $M_n$-Werte durch GPC-Analysen bestimmt werden (Hohdruck-Gel-Permeationschromatographie, Differentialrefraktometer).

Die erfindungsgemäßen Depot-Arzneimittel können noch weitere Hilfsstoffe enthalten, z.B. Lactose oder Magnesiumstearat sowie übliche Fließ- oder Gleitmittel. Die Tabletten können zur Geschmacksisolierung bevorzugt mit einem Filmlack überzogen werden, wie er z.B. in der WO 90/00391 beschrieben ist.

Es hat sich dabei herausgestellt, daß sich die erfindungsgemäßen Tabletten aufgrund ihrer Oberfläche besonders gut für eine derartige Beschichtung eignen.

Die erfindungsgemäßen Depot-Arzneimittel in Tablettenform können auf an sich bekannte Weise hergestellt werden. Die Pulver werden in einem Mischer trocken gemischt. Nach Zugabe von Wasser wird die Masse granuliert, z.B. durch Pressen durch ein Sieb, und gegebenenfalls nach Zugabe von Fließmittel zu Tabletten gepreßt. Falls erforderlich, z.B. bei bitterem Geschmack des Wirkstoffs, wird in üblicher Weise ein Filmüberzug aufgebracht.

Beispiele

Beispiel 1, Tabletten

| a) | Verapamil - HCl | 120 mg/Tab |
|---|---|---|
| | Natrium-Alginat | 120 mg/Tab |
| | Mikrokrist. Cellulose | 38 mg/Tab |
| b) | 60/40-Copolymer aus 1-Vinyl-2-pyrrolidon und Vinylacetat (Korngrößenverteilung: 15 % <50 μm, 2 % >250 μm; $M_w/M_n$=3,0 ± 0,5; $M_w$=54.000) | 20 mg/Tab |
| c) | Magnesiumstearat | 2 mg/Tab |
| | | 300 mg/Tab |

Herstellung:

1,2 kg Verapamil-HCl, 1,2 kg Natrium-Alginat und 0,38 kg Mikrokristalline Cellulose wurden homogen gemischt und in einem Feuchtkneter und mit einem Kleister von 200 g des oben genannten 60/40-Copolymeren in 1,8 kg Wasser durchfeuchtet und intensiv zu einer handfeuchten Massen granuliert; das Granulat wurde durch ein 2,5 mm Sieb getrieben und dieses gesiebte Granulat auf eine Restfeuchte von 6 % abgetrocknet. Das getrocknete Granulat wurde durch ein 1,2 mm Sieb getrieben und mit 20 g Magnesium vermischt zum fertigen Tablettiergut.

Tablettierung:

Excenter-Presse
Tablettengewicht: 300 mg
Stempel: 9 mm Durchmesser, gewölbt
Preßdruck: 15 kN
Härte der Tabletten: 37 N
Friabilität: 0,1 % Abrieb, sehr glatte Oberfläche (Roche Friabilator, 400 Umdrehungen)

Beispiel 2, Tabletten

| a) | Verapamil - HCl | 200 mg/Tab |
|---|---|---|
| | Natrium-Alginat | 400 mg/Tab |
| | Mikrokrist. Cellulose | 20 mg/Tab |
| b) | Polyvinylpyrrolidon (Korngrößenverteilung: 10 % <50 $\mu$m, 5 % >250 $\mu$m; $M_w/M_n$=2,8 ± 0,5; $M_w$=46.000) | 40 mg/Tab |
| c) | Mikrokristallne Cellulose | 20 mg/Tab |
| | Magnesiumstearat | 4 mg/Tab |
| | | 684 mg/Tab |

Herstellung analog zu Beispiel 1, gleiche Ansatzgröße; Stoffe unter a) mischen, mit b) befeuchten und intensiv mischen, 2,5 mm Sieb, trocknen, 1,2 mm Sieb und mischen mit Stoffen unter c).
Tablettierung: Oblong-Stempel 18,5 x 6,5 mm

| Preßdruck: | Härte: | Friabilität: |
|---|---|---|
| 10 kN | 80 N | 0,2 % |
| 150 kN | 110 N | 0,1 % |
| 25 kN | 180 N | 0,05 % |
| glatte Oberfläche | | |

Die Beispiele zeigen, daß der Einsatz von PVP- bzw. PVP/PVA-Präparaten zu einer sehr guten Härte der Tabletten und zu einer geringen Friabilität führt.
Friabilität ist das Maß in Gewichtsprozent für den mechanischen Abrieb der Tabletten.
Zum ordnungsgemäßen Coating von Tabletten ist eine Friabilität unter 1 % erforderlich.

**Patentansprüche**

1. Depot-Arzneimittel, enthaltend einen Wirkstoff, Alginat und Hilfsstoffe, dadurch gekennzeichnet, daß es als einen Hilfsstoff mindestens ein wasserlösliches, polymeres Bindemittel aus der Gruppe Polyvinylpyrrolidon und Vinylacetat-Vinylpyrrolidon-Copolymeres enthält, wobei das polymere Bindemittel folgende Eigenschaften aufweist:

   a) einen $M_w/M_n$-Wert von 1,5 - 5,0

   b) Korngrößenverteilung:
   Anteil < 50 µm maximal 18 %
   Anteil > 250 µm maximal 20 %.

2. Depot-Arzneimittel nach Anspruch 1, dadurch gekennzeichnet, daß es als Bindemittel Polyvinylpyrrolidon und/oder Vinylacetat-Vinylpyrrolidon-Copolymere mit einem $M_w/M_n$-Wert von 2,0 bis 4,5 enthält.

3. Depot-Arzneimittel nach Anspruch 1, dadurch gekennzeichnet, daß es als Vinylacetat-Vinylpyrrolidon-Copolymeres ein Copolymeres mit 50 bis 70 Gew.-% Vinylpyrrolidon und 30 bis 50 Gew.-% Vinylacetat enthält.

4. Depot-Arzneimittel nach Anspruch 1, dadurch gekennzeichnet, daß die Bindemittel ein Molekulargewicht $M_w$ von 5.000 bis 1.500.000, insbesondere von 10.000 bis 80.000 aufweisen.

5. Depot-Arzneimittel nach Anspruch 1, dadurch gekennzeichnet, daß es als Wirkstoff Verapamil enthält.

6. Depot-Arzneimittel nach Anspruch 1, dadurch gekennzeichnet, daß es Wirkstoff, Alginat und Bindemittel im Gewichtsverhältnis 1:0-7,3 : 0,05-2,0 enthält.